# EUROPEAN PATENT SPECIFICATION

(11) **EP 4 086 324 B1**
(45) Date of publication and mention of the grant of the patent: **26.02.2025**
(21) Application number: 20910945.3
(22) Date of filing: 03.01.2020
(51) Int. Cl.: C09K 11/67, A61K 49/00

(54) **PREPARATION METHOD FOR IN VIVO DEGRADABLE UPCONVERSION NANOMATERIAL**
VERFAHREN ZUR HERSTELLUNG VON IN VIVO ABBAUBARES HOCHKONVERSIONSNANOMATERIAL
PROCÉDÉ DE PRÉPARATION DE NANOMATÉRIAU À CONVERSION ASCENDANTE DÉGRADABLE IN VIVO

(43) Date of publication of application: 09.11.2022
(73) Proprietor: ZhongKe Rare Earth Nanotechnology (Hebei) Co., Ltd., Hebei 7200 (CN)
(72) Inventor: HONG, Maochun, Fuzhou, Fujian 350002 (CN); PENG, Pengfei, Fuzhou, Fujian 350002 (CN); LIU, Yongsheng, Fuzhou, Fujian 350002 (CN); FU, Huhui, Fuzhou, Fujian 350002 (CN)
(74) Representative: Gille Hrabal Partnerschaftsgesellschaft mbB Patentanwälte
(86) International application number: PCT/CN2020/070277
(87) International publication number: WO 2021/134786

(56) References cited:
- CN-A- 107 163 937
- CN-A- 108 359 458
- CN-A- 108 559 511
- LUO WENQIN ET AL: "Localization induced intense red upconversion luminescence in monodispersed K3ZrF7:Yb3+/Er3+ nanocrystals", CHEMICAL PHYSICS LETTERS, vol. 658, 1 August 2016 (2016-08-01), NL, pages 215 - 219, XP093004033, ISSN: 0009-2614, DOI: 10.1016/j.cplett.2016.06.041
- SONG JUN ET AL: "Lanthanide-doped Na3ZrF7upconversion nanoparticles synthesized by a facile method", JOURNAL OF ALLOYS AND COMPOUNDS, ELSEVIER SEQUOIA, LAUSANNE, CH, vol. 658, 7 November 2015 (2015-11-07), pages 914 - 919, XP029369893, ISSN: 0925-8388, DOI: 10.1016/J.JALLCOM.2015.11.026
- FU HU-HUI, LIU YONG-SHENG;JIANG FEI-LONG;HONG MAO-CHUN: "Controlled Synthesis and Optical Properties of Lanthanide-doped Na3ZrF7 Nanocrystals", JIEGOU HUAXUE - CHINESE JOURNAL OF STRUCTURAL CHEMISTRY, ZHONGGUO KEXUEYUAN FUJIAN WUZHI JIEGOU YANJIUSUO, CN, vol. 37, no. 11, 1 November 2018 (2018-11-01), CN, pages 1737 - 1748, XP055827829, ISSN: 0254-5861, DOI: 10.14102/j.cnki.0254-5861.2011-2028

## Description

### TECHNICAL FIELD

The present disclosure relates to the technical field of luminescent materials, and particularly to an *in vivo* biodegradable rare earth-doped zirconium/hafnium-based alkali metal fluoride upconversion luminescent nanomaterial, a method for preparing the same and use thereof.

### BACKGROUND

Rare earth-doped upconversion inorganic nanomaterials, having characteristics of low toxicity, good photobleaching resistance, no background fluorescence, deeper optical penetration depth and the like, are promising fluorescent contrast agents, and exhibit excellent prospect in various *in vivo* applications such as biological detection, imaging, and disease diagnosis and treatment. However, existing rare earth-doped upconversion inorganic nanomaterials, represented by the most representative β-NaYF₄, are not biodegradable *in vivo.* They may accumulate in large quantities in *vivo,* and cannot be effectively eliminated *in vivo* in a harmless manner, which make their clinical transformation almost impossible.

Therefore, there is an urgent need for an *in vivo* biodegradable rare earth-doped upconversion luminescent nanomaterial. Luo Wenqin et al, Chemical Physics Letters, vol. 658, 1 August 2016 (2016-08-01), pages 215-219 discloses a method for preparing a luminescent material of formula K3ZrF7:Yb,Er.

### SUMMARY

The present disclosure is intended to provide an *in vivo* biodegradable rare earth-doped zirconium/hafnium-based alkali metal fluoride upconversion luminescent nanomaterial, a method for preparing the same and use thereof.

For the above purpose, according to one aspect of the present disclosure, provided is an *in vivo* biodegradable upconversion inorganic nanomaterial, obtained from a rare earth-doped zirconium/hafnium-based alkali metal fluoride matrix material, wherein the matrix material has a general formula of MₓT_{y}F_{x+4y}, and has a rare earth-doped nanocrystal with a general structural formula of MₓT_{y}F_{x+4y}:z% Ln, wherein M is one or more of Li, Na and K; T is Zr and/or Hf; Ln is one or more selected from Yb, Er, Tm, Ho, Gd, Eu, Tb, Sm, Dy, Ce and Nd; 1 ≤ x ≤ 7; 1 ≤ y ≤ 6; 0 ≤ z ≤ 50.

Furthermore, the rare earth-doped nanocrystal described herein has a general structural formula of MₓT_{y}F_{x+4y}:z% Ln, wherein 1 ≤ x ≤ 3; 1 ≤ y ≤ 2; 10.5 ≤ z ≤ 22. Preferably, 21 ≤ z ≤ 22. Furthermore, the rare earth-doped nanocrystal described herein has a general structural formula of MₓT_{y}F_{x+4y}:z% Ln, wherein x = 3, y = 1, the matrix material is K₃ZrF₇, and the rare earth-doped nanocrystal described herein has a general structural formula of K₂ZrF₇:z% Ln.

Furthermore, the rare earth-doped nanocrystal has a general structural formula of MₓT_{y}F_{x+4y}:z% Ln, wherein x = 3, y = 1, the matrix material is K₃ZrF₇, and the rare earth-doped nanocrystal described herein has a general structural formula of K₂ZrF₇:z1% Yb/z2% Er, wherein 5 ≤ z1 ≤ 30, and 0.5 ≤ z2 ≤ 5. Preferably, 10 ≤ z1 ≤ 20, and 0.5 ≤ z2 < 2.

Furthermore, the doped nanocrystal has a structural formula of KₓZr_{y}F_{x+4y}:20% Yb/2% Er, KₓZr_{y}F_{x+4y}:20% Yb/1% Tm, KₓHf_{y}F_{x+4y:}20% Yb/2% Er, KₓZr_{y1}Hf_{y2}F_{x+4(y1+y2)}:20% Yb/2% Er, NaₓZr_{y}F_{x+4y}:20% Yb/2% Er, NaₓHf_{y}F_{x+4y}:20% Yb/2% Er, NaₓZr_{y1}Hf_{y2}F_{x+4(y1+y2)}:20% Yb/2% Er and LiₓZr_{y}F_{x+4y}:20% Yb/2% Er, wherein 1 ≤ y1 + y2 ≤ 6.

Preferably, the rare earth-doped nanocrystal is K₃ZrF₇:20% Yb/2% Er, K₂ZrF₆:20% Yb/2% Er, KZrF₅:20% Yb/2% Er, K₃HfF₇:20% Yb/2% Er, K₂HfF₆:20% Yb/2% Er, K₃Zr_{0.5}Hf_{0.5}F₇:20% Yb/2% Er, Na₃ZrF₇:20% Yb/2% Er, Na₂ZrF₆:20% Yb/2% Er, Na₅Zr₂F₁₃:20% Yb/2% Er, Na₇Zr₆F₃₁:20% Yb/2% Er, Na₃HfF₇:20% Yb/2% Er, Na₅Hf₂F₁₃:20% Yb/2% Er, Na₃Zr_{0.5}Hf_{0.5}F₇:20% Yb/2% Er, Li₄ZrF₈:20% Yb/2% Er and Li₂ZrF₆:20% Yb/2% Er.

According to another aspect of the present disclosure, provided is a method for preparing the in *vivo* biodegradable upconversion inorganic nanomaterial, comprising: subjecting a salt containing zirconium/hafnium ion, rare earth acetate, ammonium fluoride and alkali metal hydroxide as starting materials to a high temperature solvent coprecipitation process to give the rare earth-doped zirconium/hafnium-based alkali metal fluoride upconversion nanomaterial.

Furthermore, the high temperature solvent coprecipitation process comprises the following steps of: S1, weighing, mixing and adding zirconium/hafnium acetylacetonate and rare earth acetate to a solvent to give a solid mixture; S2, heating and incubating the solid mixture to dissolve reactants in the solid mixture to give a mixed solution; S3, adding a solution of ammonium fluoride and alkali metal hydroxide in methanol to the mixed solution obtained in the step S2, and heating and incubating to remove methanol and water; and S4, continuously heating and incubating, cooling to room temperature, precipitating, separating, washing and drying to give the rare earth-doped zirconium/hafnium-based alkali metal fluoride upconversion nanomaterial.

Furthermore, the salt containing zirconium/hafnium ion is one or more selected from zirconium/hafnium acetylacetonate, zirconium/hafnium acetate, zirconium/hafnium chloride, zirconium/hafnium nitrate and zirconium/hafnium oxychloride. The rare earth ion in the rare earth acetate is one or more selected from Yb, Er, Tm, Ho, Gd, Eu, Tb, Sm, Dy, Ce and Nd. Preferably, a ratio of zirconium/hafnium ion to the rare earth metal salt is (1-10):1, preferably (2-5):1. More preferably, the rare earth acetate is a mixture of ytterbium acetate and erbium acetate, and a molar ratio of the rare earth acetate to zirconium/hafnium acetylacetonate is 1:4.

Preferably, the solvent is a mixed solvent of oleic acid and octadecene. Preferably, a volume ratio of oleic acid to octadecene is 1:(0.5-3); more preferably, the volume ratio of oleic acid to octadecene is 1:2.

A molar ratio of ammonium fluoride to the alkali metal hydroxide is 7:(6-12), and preferably, the molar ratio of ammonium fluoride to the alkali metal hydroxide is 7:6.

Furthermore, the step S2 comprises: in an inert gas atmosphere, heating the solid mixture to 120-160 °C and incubating for 30-40 min, and more preferably, in an inert atmosphere, heating to 140 °C to completely dissolve the solid mixture and then naturally cooling to room temperature to give a clear solution;
Preferably, the step S3 comprises: in an inert gas atmosphere, heating to 50-70 °C and incubating for 30 min to remove methanol, and heating to 100-110 °C and incubating for 10-20 min to remove water.

Preferably, the step S4 comprises: in an inert gas atmosphere, continuously heating to 300-310 °C and incubating for 40-60 min. More preferably, the step S4 comprises: in an inert gas atmosphere, heating the mixture to 305 °C, incubating for 40 min and naturally cooling to room temperature. Preferably, the separating is centrifugation.

According to another aspect of the present disclosure, provided is use of an *in vivo* biodegradable upconversion inorganic nanomaterial in the field of biotechnology, preferably, in safe, *in vivo* biodegradable fluorescent labeling.

Furthermore, the use of the *in vivo* biodegradable upconversion inorganic nanomaterial is for acidity-dependent hydrolysis, preferably for specific labeling a tumor tissue of weak acidity. Beneficial Effects of Present Disclosure:
The present disclosure uses an inorganic zirconium/hafnium-based alkali metal fluoride system as the matrix material, and dopes upconversion rare earth ions into crystal lattices using a solvothermal synthesis process, i.e., a high temperature solvent coprecipitation process is used to prepare an upconversion inorganic luminescent nanomaterial which can be effectively biodegraded and eliminated *in vivo* having the following advantages:
1) The synthesis method features easily controlled conditions and good repeatability, the prepared nanocrystal material has good dispersibility, uniformity and repeatability, and the doping of rare earth ions realizes the upconversion luminescence under the excitation of near infrared light.
2) The nanocrystal material can be decomposed into water-soluble fluozirconate/fluohafnate ion ([T_{y}F_{x+4y}]^{x-}, wherein T is Zr or Hf), and provides a unique acidity-dependent hydrolysis capability. It can also provide upconversion luminescence while being decomposed in an aqueous environment. Due to the unique hydrolysis characteristics of the matrix material in an aqueous environment, the nanocrystal material can be hydrolyzed and eliminated quickly *in vivo,* and provides possibility for use *in vivo.*
3) The decomposition of the nanocrystal material prepared according to the present disclosure will be inhibited to a certain extent in a tumor environment of weak acidity (pH 5-6). Due to the delayed hydrolysis of the material in an environment of weak acidity, it can be present in a tumor environment of weak acidity for a longer period than in other *in vivo* environments, and can be used in specific fluorescent labeling in tumors of weak acidity.
4) The nanocrystal material prepared according to the present disclosure exhibits lower cytotoxicity and biotoxicity and good biological safety, and can be safely used *in vivo,* and is less prone to accumulation in large quantities *in vivo.* Therefore, the nanocrystal material can be used as an *in vivo* upconversion fluorescent labeling material and has potential application prospects in the fields of biological imaging, fluorescent labeling and the like.

### BRIEF DESCRIPTION OF THE DRAWINGS

FIG. 1 illustrates an X-ray powder diffraction pattern of K₃ZrF₇:20% Yb/2% Er upconversion nanocrystal according to Example 1 of the present disclosure.
FIG. 2a illustrates transmission electron micrographs of the K₃ZrF₇:20% Yb/2% Er upconversion nanocrystal according to Example 1 of the present disclosure at different resolutions;
FIG. 2b illustrates the particle size distribution of nanoparticles of the K₃ZrF₇:20% Yb/2% Er upconversion nanocrystal according to Example 1 of the present disclosure.
FIG. 3 illustrates an upconversion emission spectrum of the K₃ZrF₇:20% Yb/2% Er upconversion nanocrystal according to Example 1 of the present disclosure under excitation of a low power density 980 nm continuous semiconductor laser.
FIG. 4 illustrates an X-ray powder diffraction pattern of K₃HfF₇:20% Yb/2% Er upconversion nanocrystal according to Example 2 of the present disclosure.
FIG. 5a illustrates transmission electron micrographs of the K₃HfF₇:20% Yb/2% Er upconversion nanocrystal according to Example 2 of the present disclosure at different resolutions;
FIG. 5b illustrates the particle size distribution of nanoparticles of the K₃HfF₇:20% Yb/2% Er upconversion nanocrystal according to Example 2 of the present disclosure.
FIG. 6 illustrates an upconversion emission spectrum of the K₃HfF₇:20% Yb/2% Er upconversion nanocrystal according to Example 2 of the present disclosure under excitation of a low power density 980 nm continuous semiconductor laser.
FIG. 7 illustrates a curve of up-conversion luminous intensity versus time under excitation of 980 nm laser and a corresponding luminescence photograph after adding deionized water to a solution of the K₃ZrF₇:20% Yb/2% Er up-conversion nanocrystal according to Example 1 of the present disclosure in cyclohexane.
FIG. 8 illustrates a curve of up-conversion luminous intensity versus time under excitation of 980 nm laser and a corresponding luminescence photograph after adding a powder of the K₃ZrF₇:20% Yb/2% Er up-conversion nanocrystal according to Example 1 of the present disclosure to deionized water.
FIG. 9 illustrates a curve of up-conversion luminous intensity versus time under excitation of 980 nm laser and a corresponding luminescence photograph after adding a powder of the K₃ZrF₇:20% Yb/2% Er up-conversion nanocrystal according to Example 1 of the present disclosure to strong acids and strong bases.
FIG. 10 illustrates a curve of up-conversion luminous intensity versus time under excitation of 980 nm laser and a corresponding luminescence photograph after adding a powder of the K₃ZrF₇:20% Yb/2% Er up-conversion nanocrystal according to Example 1 of the present disclosure to an aqueous solution of weak basicity.
FIG. 11 illustrates a curve of up-conversion luminous intensity versus time under excitation of 980 nm laser and a corresponding luminescence photograph after adding a powder of the K₃ZrF₇:20% Yb/2% Er up-conversion nanocrystal according to Example 1 of the present disclosure to an aqueous solution of weak acidity.
FIG. 12 illustrates a CCD imaging system used in tests of the present disclosure.
FIG. 13 is a schematic illustrating the effect of the K₃ZrF₇:20% Yb/2% Er upconversion nanocrystal according to Example 1 of the present disclosure in subcutaneous imaging of nude mice.
FIG. 14 illustrates the results of a cytotoxicity test for K₃ZrF₇:20% Yb/2% Er upconversion nanocrystal according to Example 1 of the present disclosure.
FIG. 15 illustrates a curve of body weight of mice versus time in a mouse acute toxicity test of the K₃ZrF₇:20% Yb/2% Er upconversion nanocrystal according to Example 1 of the present disclosure.
FIG. 16 illustrates tissue slices of major organs in mice after the mouse acute toxicity test of the K₃ZrF₇:20% Yb/2% Er upconversion nanocrystal according to Example 1 of the present disclosure.
FIG. 17 illustrates muscle tissue slices after a rat muscle stimulation test of the K₃ZrF₇:20% Yb/2% Er upconversion nanocrystal according to Example 1 of the present disclosure.
FIG. 18 illustrates the distribution of elements in various organs in mice within a short period of time after an injection in tail vein of the K₃ZrF₇:20% Yb/2% Er upconversion nanocrystal according to Example 1 of the present disclosure.

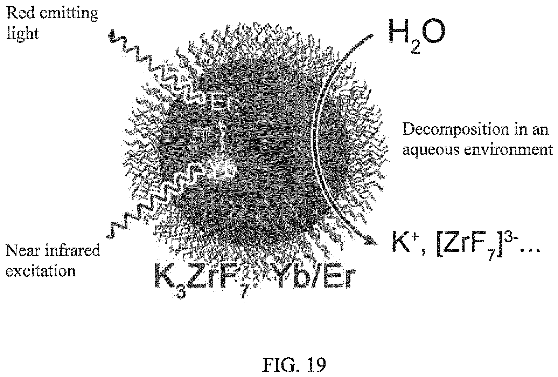

FIG. 19 illustrates a schematic of the conversion principle of the K₃ZrF₇:20% Yb/2% Er nanocrystal according to Example 1 of the present disclosure.

### DETAILED DESCRIPTION

The preparation method disclosed herein will be further illustrated in detail with reference to the following specific examples. It should be understood that the following examples are merely exemplary illustration and explanation of the present disclosure, and should not be construed as limiting the protection scope of the present disclosure. All techniques implemented based on the above content of the present disclosure are encompassed within the protection scope of the present disclosure.

According to the present disclosure, provided is an *in vivo* biodegradable upconversion inorganic nanomaterial, obtained from a rare earth-doped zirconium/hafnium-based alkali metal fluoride matrix material, wherein the matrix material has a general formula of MₓT_{y}F_{x+4y}, and has a rare earth-doped nanocrystal with a general structural formula of MₓT_{y}F_{x+4y}:z% Ln, wherein M is one or more of Li, Na and K; T is Zr and/or Hf; Ln is one or more selected from Yb, Er, Tm, Ho, Gd, Eu, Tb, Sm, Dy, Ce and Nd; 1 ≤ x ≤ 7; 1 ≤ y ≤ 6; 0 ≤ z ≤ 50.

Preferably, in the general structural formula of the nanocrystal, 1 ≤ x ≤ 3; 1 ≤ y ≤ 2; 10.5 < z ≤ 22. More preferably, 21 ≤ z ≤ 22.

The present disclosure uses the rare earth-doped zirconium/hafnium-based alkali metal fluoride, wherein fluozirconate/fluohafnate radical ions in the matrix material provide unique capability of acidity-dependent hydrolysis, and doping of rare earth ions realizes the upconversion luminescence under the excitation of near infrared light, such that it can be better used for detection *in vivo.*

When the powder of the rare earth-doped zirconium/hafnium-based alkali metal fluoride nanomaterial prepared in the present disclosure is immersed in an aqueous environment, hydrolysis occurs rapidly without other conditions, and the product after an evaporation of the supernatant is still the original matrix material as confirmed by detection. The nanomaterial can also be dispersed in solvents such as cyclohexane, olive oil and peanut oil, from which it can be diffused into an external aqueous environment and hydrolyzed to give a hydrolysate without luminescence.

According to the present disclosure, the rare earth-doped zirconium/hafnium-based alkali metal fluoride nanomaterial is hydrolyzed more quickly in environments of strong acidity and strong basicity, while the decomposition thereof will be inhibited to a certain extent when the nanomaterial is in an environment of weak acidity (pH 5-6), exhibiting a reduced hydrolysis rate. Taking advantage of the slow hydrolysis in an environment of weak acidity, the nanomaterial can be used for *in vivo* fluorescent labeling in a tumor environment of weak acidity.

According to the present disclosure, in the structural formula of the rare earth-doped zirconium/hafnium-based alkali metal fluoride upconversion inorganic nanomaterial, x = 3, y = 1, the matrix material is K₃ZrF₇, and the rare earth-doped nanocrystal has a general structural formula of K₃ZrF₇:z% Ln.

According to the present disclosure, in the structural formula of the rare earth-doped zirconium/hafnium-based alkali metal fluoride upconversion inorganic nanomaterial, x = 3, y = 1, the matrix material is K₃ZrF₇, and the rare earth-doped nanocrystal has a general structural formula of K₃ZrF₇:z1% Yb/z2% Er, wherein 5 ≤ z1 ≤ 30, and 0.5 ≤ z2 ≤ 5. Preferably, 10 ≤ z1 ≤ 20, and 0.5 ≤ z2 ≤ 2.

In one specific embodiment of the present disclosure, the rare earth-doped nanocrystal has a structural formula of KₓZr_{y}F_{x+4y}:20% Yb/2% Er, KₓZr_{y}F_{x+4y}:20% Yb/1% Tm, KₓHf_{y}F_{x+4y}:20% Yb/2% Er, KₓZr_{y}Hf_{y2}F_{x+4(y1+y2)}:20% Yb/2% Er, NaₓZr_{y}F_{x+4y}:20% Yb/2% Er, NaₓHf_{y}F_{x+4y}:20% Yb/2% Er, NaZr_{y1}Hf_{y2}F_{x+4(y1+y2)}:20% Yb/2% Er and LiₓZr_{y}F_{x+4y}:20% Yb/2% Er, wherein 1 ≤ yl + y2 ≤ 6.

Specifically, the structural formula of the nanocrystal can be K₃ZrF₇:20% Yb/2% Er, K₂ZrF₆:20% Yb/2% Er, KZrF₅:20% Yb/2% Er, K₃HfF₇:20% Yb/2% Er, K₂HfF₆:20% Yb/2% Er, K₃Zr_{0.5}Hf_{0.5}F₇:20% Yb/2% Er, Na₃ZrF₇:20% Yb/2% Er, Na₂ZrF₆:20% Yb/2% Er, Na₅Zr₂F₁₃:20% Yb/2% Er, Na₇Zr₆F₃₁:20% Yb/2% Er, Na₃HfF₇:20% Yb/2% Er, Na₅Hf₂F₁₃:20% Yb/2% Er, Na₃Zr_{0.5}Hf_{0.5}F₇:20% Yb/2% Er, Li₄ZrF₈:20% Yb/2% Er, or Li₂ZrF₆:20% Yb/2% Er.

According to another aspect of the present disclosure, provided is a method for preparing the *in vivo* biodegradable upconversion inorganic nanomaterial, comprising: subjecting a salt containing zirconium/hafnium ion, rare earth acetate, ammonium fluoride and alkali metal hydroxide as starting materials to a high temperature solvent coprecipitation process to give the rare earth-doped zirconium/hafnium-based alkali metal fluoride upconversion nanomaterial.

Preferably, the high temperature solvent coprecipitation process comprises the following steps of: S1, weighing, mixing and adding zirconium/hafnium acetylacetonate and rare earth acetate to a solvent to give a solid mixture; S2, heating and incubating the solid mixture to dissolve reactants in the solid mixture to give a mixed solution; S3, adding a solution of ammonium fluoride and alkali metal hydroxide in methanol to the mixed solution obtained in the step S2, and heating and incubating to remove methanol and water; and S4, continuously heating and incubating, cooling to room temperature, precipitating, separating, washing and drying to give the rare earth-doped zirconium/hafnium-based alkali metal fluoride upconversion nanomaterial.

In the present disclosure, the salt containing zirconium/hafnium ion is one or more selected from zirconium/hafnium acetylacetonate, zirconium/hafnium acetate, zirconium/hafnium chloride, zirconium/hafnium nitrate and zirconium/hafnium oxychloride. In the present disclosure, the salt containing zirconium/hafnium ion is preferably selected from, but is not limited to those above. Salts are suitable as long as they produce water-soluble zirconium/hafnium ions.

Preferred rare earth ion in the rare earth acetate is one or more selected from ytterbium (Yb), erbium (Er), thulium (Tm), holmium (Ho), gadolinium (Gd), europium (Eu), terbium (Tb), samarium (Sm) dysprosium (Dy), cerium (Ce) and neodymium (Nd). Preferably, ytterbium (Yb) and erbium (Er) are co-doped. Preferably, a molar ratio of zirconium/hafnium ion to the rare earth metal salt is (1-10):1, preferably (2-5):1.

More preferably, the rare earth acetate is a mixture of ytterbium acetate and erbium acetate, wherein a molar ratio of the rare earth acetate to zirconium/hafnium acetylacetonate is 1:4.

Preferably, the solvent is a mixed solvent of oleic acid and octadecene. Preferably, a volume ratio of oleic acid to octadecene is 1:(0.5-3), more preferably 1:2. According to the present disclosure, a molar ratio of ammonium fluoride to the alkali metal hydroxide is 7:(6-12), and preferably, the molar ratio of ammonium fluoride to the alkali metal hydroxide is 7:6.

According to the present disclosure, the step S2 comprises: in an inert gas atmosphere, heating the solid mixture to 120-160 °C and incubating for 30-40 min. By heating and incubating, the solid can be completely dissolved to give a clear solution. More preferably, the step comprises: in an inert atmosphere, heating to 140 °C to completely dissolve the solid mixture and then naturally cooling to room temperature to give a clear solution.

Preferably, the step S3 comprises: in an inert gas atmosphere, heating to 50-70 °C and incubating for 30 min to remove methanol, and heating to 100-110 °C and incubating for 10-20 min to remove water.

Preferably, the step S4 comprises: in an inert gas atmosphere, continuously heating to 300-310 °C and incubating for 40-60 min. More preferably, the step S4 comprises: in an inert gas atmosphere, heating the mixture to 305 °C, incubating for 40 min and naturally cooling to room temperature. Centrifugation is preferred in the present disclosure.

The present disclosure further provides use of an *in vivo* biodegradable rare earth-doped zirconium/hafhium-based alkali metal fluoride upconversion inorganic nanomaterial in the field of biotechnology, preferably in safe, *in vivo* biodegradable fluorescent labeling.

More preferably, the use of the *in vivo* biodegradable rare earth-doped zirconium/hafnium-based alkali metal fluoride upconversion inorganic nanomaterial is for acidity-dependent hydrolysis, preferably for specific labeling a tumor tissue of weak acidity.

### Comparative Example 1

### Preparation of β-NaYF₄:20% Yb/2% Er upconversion nanocrystal

0.2075 g of yttrium acetate, 0.07 g of ytterbium acetate and 0.007 g of erbium acetate were weighed and transferred into a two-necked flask at room temperature. 6 mL of oleic acid and 15 mL of octadecene were added as solvents. In an inert gas atmosphere, the mixture was heated to 140 °C to completely dissolve the above solids. The mixture was then naturally cooled to room temperature to give a clear solution. A solution of 0.1482 g of ammonium fluoride and 0.10 g of sodium hydroxide in 8 mL of methanol was added to the above clear solution. In an inert gas atmosphere, the mixture was heated to 50 °C and incubated until methanol was completely removed, and was then heated to 100-110 °C and incubated until water was completely removed. The mixture was heated to 300 °C and incubated for 60 min in an inert gas atmosphere, naturally cooled to room temperature, precipitated and washed to give the β-NaYF₄:20% Yb/2% Er nanocrystal.

### Example 1

### Preparation of nontoxic in vivo biodegradable K₃ZrF₇:20% Yb/2% Er upconversion nanocrystal by high temperature solvent coprecipitation

0.1961 g of zirconium acetylacetonate, 0.034 g of ytterbium acetate and 0.0034 g of erbium acetate were weighed and transferred into a two-necked flask at room temperature (wherein the molar ratio of ytterbium acetate or erbium acetate to zirconium acetylacetonate was 1:4). 8.5 mL of oleic acid and 17 mL of octadecene were added as solvents. In an inert gas atmosphere, the mixture was heated to 140 °C and incubated for 30 min to completely dissolve the above solids. The mixture was naturally cooled to room temperature to give a clear solution.

A solution of 0.1296 g of ammonium fluoride in 9 mL of methanol and a solution of 0.1683 g of potassium hydroxide in 3 mL of methanol were added to the above clear solution. In an inert gas atmosphere, the mixture was heated to 50 °C and incubated for 30 min until methanol was completely removed, and was then heated to 100-110 °C and incubated for 10-20 min until water was completely removed. In an inert gas atmosphere, the mixture was heated to 305 °C, incubated for 40 min, naturally cooled to room temperature, precipitated and washed to give a cubic K₃ZrF₇:20% Yb/2% Er nanocrystal.

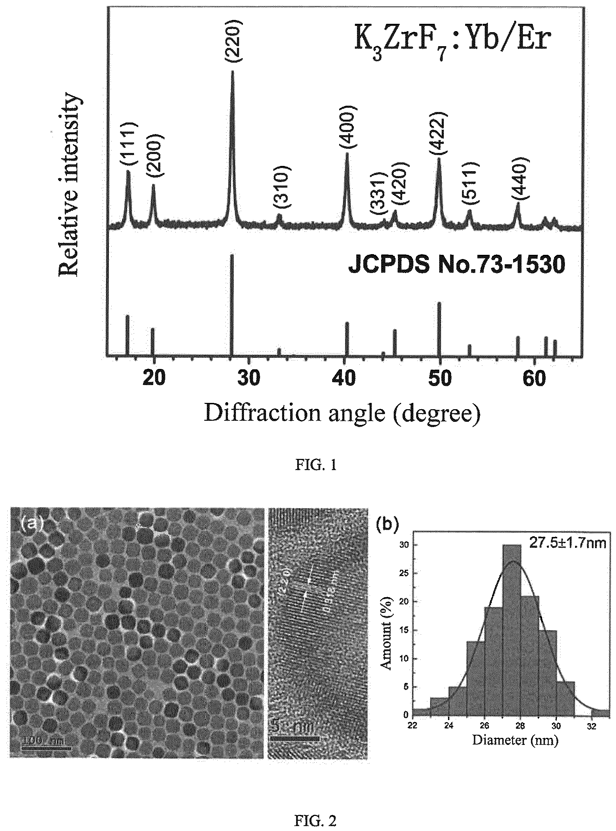

The nanocrystal obtained in Example 1 was subjected to an X-ray diffraction pattern analysis with a copper radiation wavelength of X = 0.154187 nm (MiniFlex2 X-ray diffractometer, manufactured by Rigaku). As can be seen from FIG. 1, the nanocrystal exhibited good crystallinity, and positions and relative intensities of diffraction peaks consistent with those in the PDF (Powder Diffraction File) standard card of K₃ZrF₇ (JCPDS No. 73-1530), suggesting a cubic crystal system.

FIGs. 2a and 2b are transmission electron micrograph and particle size distribution pattern of the nanocrystal obtained in Example 1 at different resolutions, respectively (TECNAI G2 F20 transmission electron microscope, manufactured by FEI). As can be seen from FIGs. 2a and 2b, the nanocrystal exhibited good dispersibility, uniform morphology, and a particle size of about 27.5 nm.

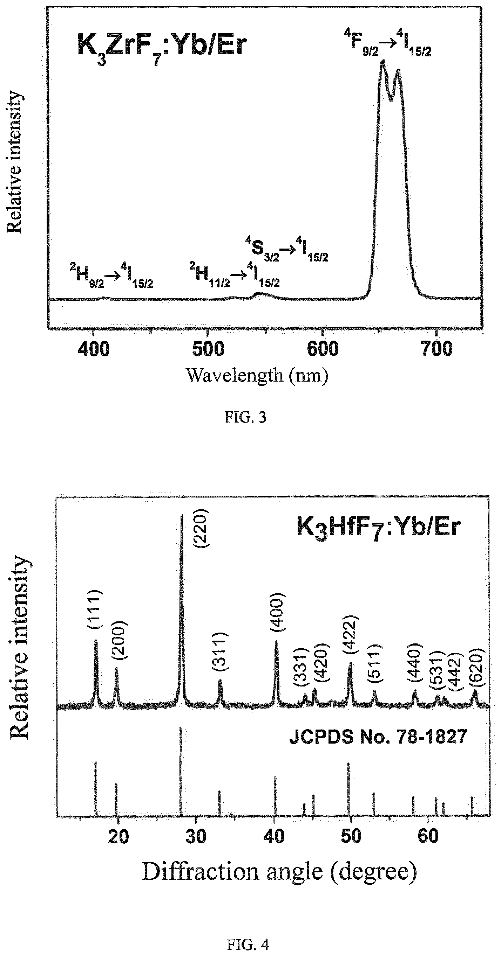

The nanocrystal in Example 1 was analyzed using a steady state transient fluorescence spectrometer (FLS980, manufactured by Edinburgh). As shown in FIG. 3, under the excitation of 980 nm light source, the oil-soluble nanocrystal emitted red upconversion light with a main peak at 656 nm, which corresponded to 4F9/2-4I15/2 electric dipole transition of Er³⁺ ion.

### Example 2

Preparation of nontoxic *in vivo* biodegradable K₃HfF₇:20% Yb/2% Er upconversion nanocrystal by high temperature solvent coprecipitation

0.2311 g of hafnium acetylacetonate, 0.034 g of ytterbium acetate and 0.0034 g of erbium acetate were weighed and transferred into a two-necked flask at room temperature. 8.5 mL of oleic acid and 17 mL of octadecene were added as solvents. In an inert gas atmosphere, the mixture was heated to 160 °C and incubated for 30 min to completely dissolve the above solids. The mixture was naturally cooled to room temperature to give a clear solution.

A solution of 0.1296 g of ammonium fluoride in 9 mL of methanol and a solution of 0.1683 g of potassium hydroxide in 3 mL of methanol were added to the above clear solution. In an inert gas atmosphere, the mixture was heated to 50 °C and incubated for 30 min until methanol was completely removed, and was then heated to 100-110 °C and incubated for 10-20 min until water was completely removed. In an inert gas atmosphere, the mixture was heated to 310 °C, incubated for 40 min, naturally cooled to room temperature, precipitated and washed to give a cubic K₃HfF₇:20% Yb/2% Er nanocrystal.

As can be seen from FIG. 4, the nanocrystal exhibited good crystallinity, and positions and relative intensities of diffraction peaks consistent with those in the PDF (Powder Diffraction File) standard card of K₃HfF₇ (JCPDS No. 78-1827), suggesting a cubic crystal system.

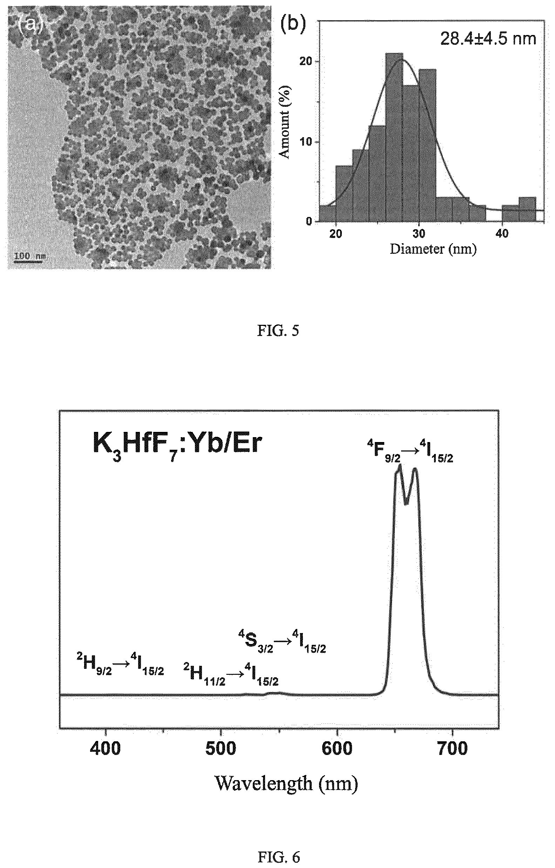

FIGs. 5a and 5b are transmission electron micrograph and particle size distribution pattern of the nanocrystal obtained in Example 2 at different resolutions, respectively (TECNAI G2 F20 transmission electron microscope, manufactured by FEI). As can be seen from FIGs. 5a and 5b, the nanocrystal exhibited a particle size of about 28.4 nm.

The nanocrystal in Example 2 was analyzed using a steady state transient fluorescence spectrometer (FLS980, manufactured by Edinburgh). As shown in FIG. 6, under the excitation of 980 nm light source, the oil-soluble nanocrystal emitted red upconversion light with a main peak at 656 nm, which corresponded to 4F9/2-4I15/2 electric dipole transition of Er³⁺ ion.

### Example 3

### Detection of hydrolysis performance of K₃ZrF₇:20% Yb/2% Er nanocrystal

The nanocrystal in Example 1 was analyzed using a steady state transient fluorescence spectrometer (FLS980, manufactured by Edinburgh). The camera was EOS 5D manufactured by Canon.

### (1) Hydrolysis of the nanocrystal in cyclohexane

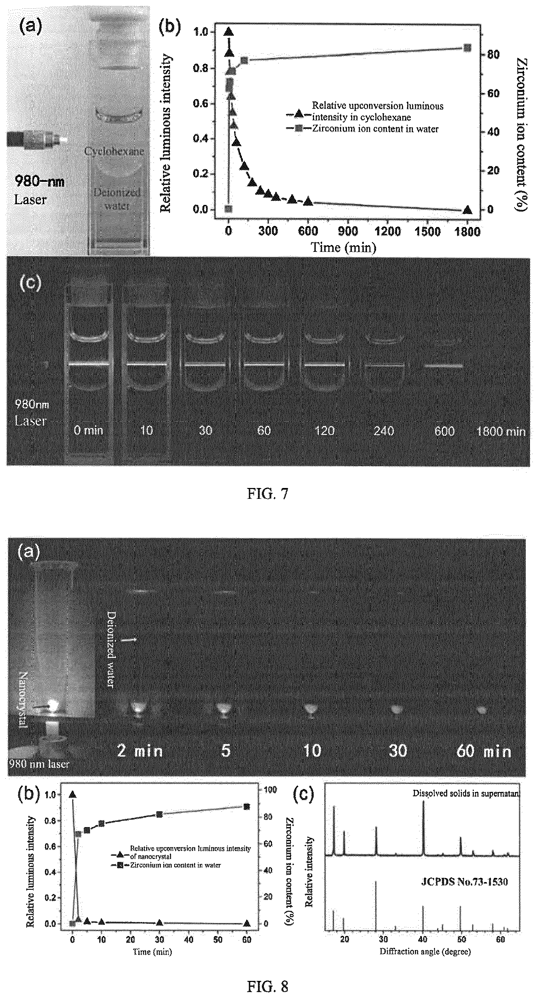

As shown in panels (a) and (c) in FIG. 7, the nanocrystal obtained in Example 1 was dissolved in cyclohexane. An equal volume of deionized water was added. Under the excitation of 980 nm laser, a gradually diminishing red emitting light was observed in the cyclohexane solution in upper layer, and no emitting light was found in the lower layer. In different time periods, the luminous intensity in upper layer was measured, and deionized water in the lower layer was detected for ions therein. As shown in (b) in FIG. 7, the relative luminous intensity of the cyclohexane solution in the upper layer gradually diminished as the material was gradually dissolved in deionized water. In summary, the K₃ZrF₇:20% Yb/2% Er nanocrystal prepared in Example 1 has good hydrolysis performance.

### (2) Decomposition of the nanocrystal powder in deionized water

As shown in panel (a) in FIG. 8, the nanocrystal prepared in Example 1 was dried to give a powder. The powder was immersed in a certain amount of deionized water. Under the excitation of 980 nm laser, the red emitting light of the solid powder gradually diminished over time, and no emitted light was observed in the supernatant. In different time periods, the luminous intensity of the solid and the content of ions in the supernatant were measured, and the luminescence photographs were recorded. As shown in panel (b) in FIG. 8, when the powder was immersed in deionized water, the relative luminous intensity rapidly diminished until substantially no emitting light was detected, and ions in the lower layer were almost completely dissolved in deionized water in the upper layer. Panels (c) in FIG. 8 is a powder diffraction pattern of the solid after immersion in the supernatant for a certain period of time and evaporation, illustrating consistent positions of diffraction peaks with those in the PDF standard card of K₃ZrF₇ (JCPDS No. 73-1530). This suggested that the nanocrystal prepared in Example 1 can be decomposed well in deionized water.

### (3) Decomposition of the nanocrystal powder in strong acid and strong base

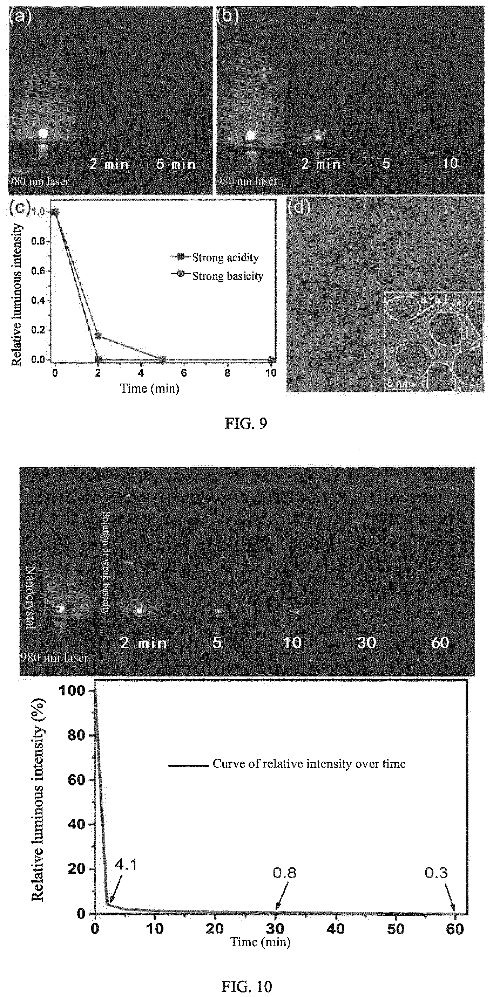

As shown in panels (a), (b) and (c) in FIG. 9, the nanocrystal prepared in Example 1 was dried to give a powder. The powder was immersed in a certain amount of strong acid (HCl, 1 mol/L) and strong base (NaOH, 3 mol/L). Under the excitation of 980 nm laser, it can be seen that for powders immersed in either strong acid in FIG. 9 (a) or strong base in FIG. 9 (b), the red emitting light diminished rapidly, and no emitting light in the supernatant was detected. FIG. 9 (d) is a transmission electron micrograph of the supernatant with residuals being KYb₃F₁₀ crystals having a particle size of about 5 nm. This suggested that the nanocrystal prepared in Example 1 can be decomposed in a solution of strong acidity or strong basicity.

### (4) Decomposition of the powder in an environment of weak basicity

The nanocrystal prepared in Example 1 was dried to give a powder. The powder was immersed in a certain amount of Tris-HCl (pH 8.8, 1 M) and NaHCO₃ (pH 8.3). Under the excitation of 980 nm laser, the red emitting light of the solid powder gradually diminished over time, and no emitted light was observed in the supernatant. In different time periods, the luminous intensity of the solid were measured, and the luminescence photographs were recorded. As shown in FIG. 10, when the powder was in an environment of weak basicity, the relative luminous intensity rapidly diminished until substantially no emitting light was detected.

### (5) Decomposition of the powder in an environment of weak acidity

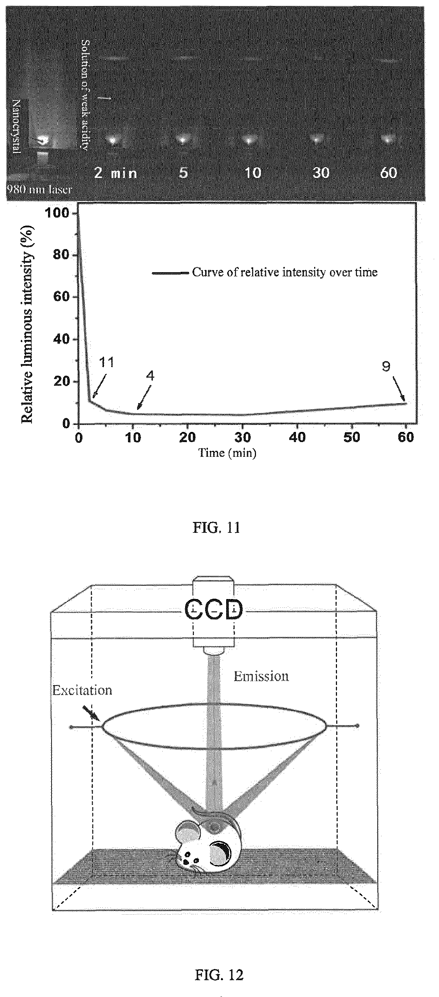

The nanocrystal prepared in Example 1 was dried to give a powder. The powder was immersed in a certain amount of NaAc-HAc (pH 5.2). Under the excitation of 980 nm laser, a reduced rate of diminution for the red emitting light in the solid powder was observed with luminescence being detected after a long period of time, and no emitted light was observed in the supernatant. In different time periods, the luminous intensity of the solid were measured, and the luminescence photographs were recorded. As shown in FIG. 11, when the powder was in an environment of weak acidity, the rate of diminution for the relative luminous intensity was relatively lower and luminescence was present over a longer period of time.

### Example 4

### Bioimaging of K₃ZrF₇:20% Yb/2% Er nanocrystal

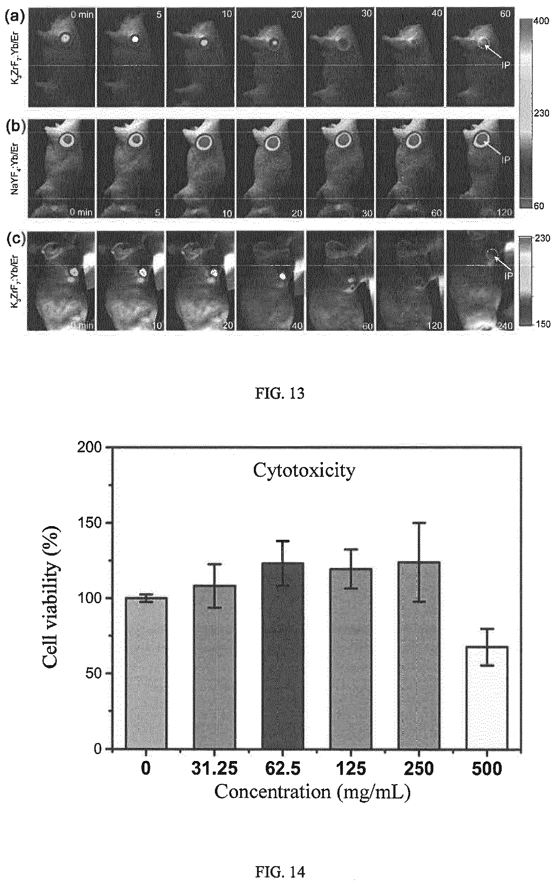

The subcutaneous imaging in nude mice was performed using a set of CCD imaging system (ANDOR) as shown in FIG. 12.
(1) Subcutaneous imaging in nude mouse: as shown in FIG. 13 (a), a small amount of a solution of the nanocrystal in Example 1 in peanut oil (50 mg/mL) was injected subcutaneously into nude mice and imaging was conducted using a CCD imaging system under excitation of 980 nm laser. Fluorescence was observed at the site of injection, which gradually diminished over time and was substantially no longer detected at about 40 min.
(2) Subcutaneous imaging in nude mouse (control group receiving NYF₄ in Comparative Example 1): as shown in FIG. 13 (b), a small amount of a solution of the NYF₄ nanocrystal in Comparative Example 1 in peanut oil (50 mg/mL) was injected subcutaneously into nude mice and imaging was conducted using a CCD imaging system under excitation of 980 nm laser. Fluorescence was observed at the site of injection with no significant changes over time, and was still detected after 2 h.
(3) Subcutaneous imaging of 4T1 tumor tissue in nude mouse: as shown in FIG. 13 (c), a small amount of a solution of the nanocrystal in Example 1 in peanut oil (50 mg/mL) was injected subcutaneously at the site of 4T1 tumors in nude mice and imaging was conducted using a CCD imaging system under excitation of 980 nm laser. Fluorescence was observed at the site of injection, which diminished over time and was substantially no longer detected at about 4 h.

This suggested that luminescence of the K₃ZrF₇:20% Yb/2% Er nanocrystal *in vivo* will diminish over time, but can be maintained for a longer period of time in a tumor environment of weak acidity.

### Example 5

### Biological safety determination of K₃ZrF₇:20% Yb/2% Er nanocrystal

### (1) Cytotoxicity test by MTT colorimetric assay using human cervical cancer HeLa cell

As shown in FIG. 14, the normal cell activity of human cervical cancer HeLa cell was maintained at a concentration range of 0-0.25 mg/mL for the nanocrystal prepared in Example 1, indicating that the nanocrystal has very low cytotoxicity.

### (2) Toxicity test of a single intravenous dose in mouse

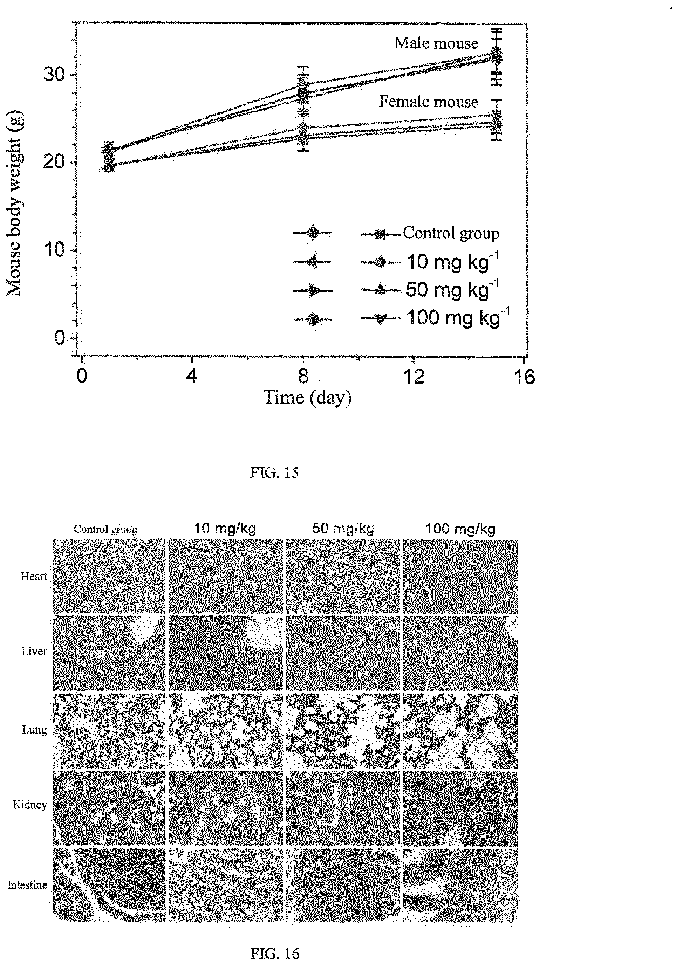

As shown in FIG. 15, in a concentration range of 0-100 mg/kg, the male and female mice exhibited no significant difference in body weight change compared with the control group, and normally matured. During the observation period, no abnormalities were observed in appearance, nervous system, respiratory and circulatory systems, genitourinary system, behavior and activity, and food and water intake. As shown in FIG. 16, no macroscopic lesions were observed in the dissection, and no abnormal lesions were observed in the heart, liver, lung, kidney and intestine tissues of all animals by histopathological examination.

### (3) Rat muscle stimulation test

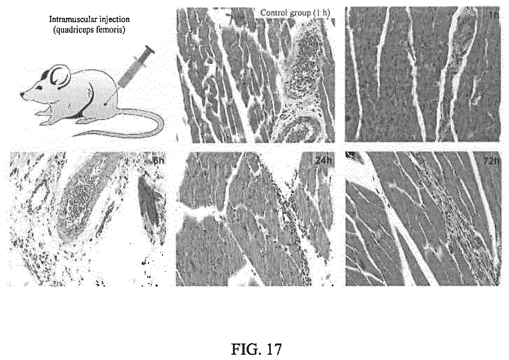

As shown in FIG. 17, the animals were euthanized at 1, 6, 24, and 72 h after administration. At the four time points, there was no significantly difference between the nanocrystal treatment arm and the control arm, and no significant local stimulation reactions such as congestion and swelling were observed.

The above tests all suggested that the K₃ZrF₇:20% Yb/2% Er nanocrystal has good biological safety.

### Example 6

### Biodegradation of K₃ZrF₇:20% Yb/2% Er nanocrystal in vivo

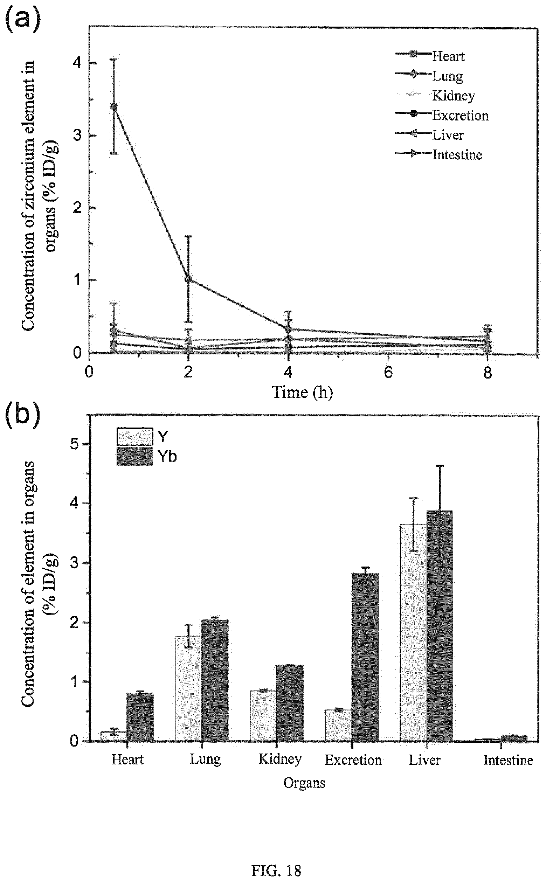

Distribution of elements *in vivo*: as shown in panel (a) in FIG. 18, a small amount of 5% aqueous DMSO solution containing the nanocrystal was injected into nude mice via tail vein, and the animal was euthanized at 0.5, 2, 4 and 8 h after administration. At the four time points, the content of zirconium element in the major organs of the nude mice was measured. It was observed that within a short period of time after injection, a large part of zirconium ions was excreted out of the body along with excretion and rarely accumulated in other major organs in the body. In contrast, as shown in FIG. 18 (b), NaYF₄ in Comparative Example 1 accumulated in the major organs over a longer period of time after injection.

Examples of the present disclosure have been described above. However, the present disclosure is not limited thereto.

## Claims

1. A method for preparing an *in vivo* biodegradable upconversion inorganic nanomaterial, comprising: subjecting a salt containing zirconium/hafnium ion, rare earth acetate, ammonium fluoride and alkali metal hydroxide as starting materials to a high temperature solvent coprecipitation process to give the rare earth-doped zirconium/hafnium-based alkali metal fluoride upconversion nanomaterial, wherein the *in vivo* biodegradable upconversion inorganic nanomaterial is derived from a rare earth-doped zirconium/hafnium-based alkali metal fluoride matrix material, wherein the matrix material has a general formula of MₓT_{y}F_{x+4y}, containing a rare earth-doped nanocrystal with a general structural formula of MₓT_{y}F_{x+4y}:z% Ln, wherein M is one or more of Li, Na and K; T is Zr and/or Hf; Ln is one or more selected from Yb, Er, Tm, Ho, Gd, Eu, Tb, Sm, Dy, Ce and Nd; 1 ≤ x ≤ 7; 1 ≤ y ≤ 6; 0 < z ≤ 50;
wherein the high temperature solvent coprecipitation process comprises the following steps of:
S1, weighing, mixing and adding zirconium/hafnium acetylacetonate and rare earth acetate to a solvent to give a solid mixture;
S2, heating and incubating the solid mixture to dissolve reactants in the solid mixture to give a mixed solution;
S3, adding a solution of ammonium fluoride and alkali metal hydroxide in methanol to the mixed solution obtained in the step S2, and heating and incubating to remove methanol and water; and
S4, continuously heating and incubating, cooling to room temperature, precipitating, separating, washing and drying to give the rare earth-doped zirconium/hafnium-based alkali metal fluoride upconversion nanomaterial.

2. The method according to claim 1, wherein the salt containing zirconium/hafnium ion is one or more selected from zirconium/hafnium acetylacetonate, zirconium/hafnium acetate, zirconium/hafnium chloride, zirconium/hafnium nitrate and zirconium/hafnium oxychloride; the rare earth ion in the rare earth acetate is one or more selected from Yb, Er, Tm, Ho, Gd, Eu, Tb, Sm, Dy, Ce and Nd;
preferably, a molar ratio of the zirconium/hafnium ion to the rare earth metal salt is(1-10): 1, preferably (2-5):1;
more preferably, the rare earth acetate is a mixture of ytterbium acetate and erbium acetate, and a molar ratio of the rare earth acetate to the zirconium/hafnium acetylacetonate is 1:4;
preferably, the solvent is a mixed solvent of oleic acid and octadecene, preferably, a volume ratio of the oleic acid to the octadecene is 1:(0.5-3), and more preferably, the volume ratio of the oleic acid to the octadecene is 1:2;
a molar ratio of the ammonium fluoride to the alkali metal hydroxide is 7:(6-12), and preferably, the molar ratio of the ammonium fluoride to the alkali metal hydroxide is 7:6.

3. The method according to claim 1, wherein the step S2 comprises: in an inert gas atmosphere, heating the solid mixture to 120-160 °C and incubating for 30-40 min,
and more preferably, in an inert atmosphere, heating to 140 °C to completely dissolve the solid mixture and then naturally cooling to room temperature to give a clear solution;
preferably, the step S3 comprises: in an inert gas atmosphere, heating to 50-70 °C and incubating for 30 min to remove methanol, and heating to 100-110 °C and incubating for 10-20 min to remove water;
preferably, the step S4 comprises: in an inert gas atmosphere, continuously heating to 300-310 °C and incubating for 40-60 min;
more preferably, the step S4 comprises: in an inert gas atmosphere, heating the mixture to 305 °C, incubating for 40 min and naturally cooling to room temperature; preferably, the separating is centrifugation.

## Patentansprüche

1. Verfahren zum Herstellen eines *in vivo* biologisch abbaubaren anorganischen Hochkonversionsnanomaterials, umfassend: Unterziehen eines Salzes, das Zirkonium-/Hafniumion, Seltenerdacetat, Ammoniumfluorid und Alkalimetallhydroxid als Ausgangsmaterialien enthält, einem Hochtemperatur-Lösungsmittel-Kopräzipitationsprozess, um das mit seltenen Erden dotierte Alkalimetallfluorid-Hochkonversionsnanomaterial auf Zirkonium-/Hafniumbasis zu ergeben, wobei das *in vivo* biologisch abbaubare anorganische Hochkonversionsnanomaterial aus einem mit seltenen Erden dotierten Alkalimetallfluorid-Matrixmaterial auf Zirkonium-/Hafniumbasis abgeleitet ist, wobei das Matrixmaterial eine allgemeine Formel MₓT_{y}F_{x+4y} aufweist, einen mit seltenen Erden dotierten Nanokristall mit einer allgemeinen Strukturformel MₓT_{y}F_{x+4y}:z% Ln enthält, wobei M eines oder mehrere von Li, Na und K ist; T Zr und/oder Hf ist; Ln eines oder mehrere ausgewählt aus Yb, Er, Tm, Ho, Gd, Eu, Tb, Sm, Dy, Ce und Nd ist; 1 ≤ x ≤ 7; 1 ≤ y ≤ 6; 0 < z ≤ 50;
wobei der Hochtemperatur-Lösungsmittel-Kopräzipitationsprozess die folgenden Schritte umfasst:
S1, Abwiegen, Mischen und Hinzufügen von Zirkonium-/Hafniumacetylacetonat und Seltenerdacetat zu einem Lösungsmittel, um eine feste Mischung zu ergeben;
S2, Erwärmen und Inkubieren der festen Mischung, um die Reaktanten in der festen Mischung aufzulösen, um eine gemischte Lösung zu ergeben;
S3, Hinzufügen einer Lösung von Ammoniumfluorid und Alkalimetallhydroxid in Methanol zu der in Schritt S2 erhaltenen gemischten Lösung und Erwärmen und Inkubieren, um Methanol und Wasser zu entfernen; und
S4, kontinuierliches Erwärmen und Inkubieren, Kühlen auf Raumtemperatur, Ausfällen, Trennen, Waschen und Trocknen, um das mit seltenen Erden dotierte Alkalimetallfluorid-Hochkonversionsnanomaterial auf Zirkonium-/Hafniumbasis zu erhalten.

2. Verfahren nach Anspruch 1, wobei das Salz, das Zirkonium-/Hafniumion enthält, eines oder mehrere ausgewählt aus Zirkonium-/Hafniumacetylacetonat, Zirkonium-/Hafniumacetat, Zirkonium-/Hafniumchlorid, Zirkonium-/Hafniumnitrat und Zirkonium-/Hafniumoxychlorid ist; das Seltenerdion im dem Seltenerdacetat eines oder mehrere ausgewählt aus Yb, Er, Tm, Ho, Gd, Eu, Tb, Sm, Dy, Ce und Nd ist;
wobei vorzugsweise ein Molverhältnis des Zirkonium-/Hafniumions zu dem Seltenerdmetallsalz (1-10):1, vorzugsweise (2-5):1 beträgt;
wobei das Seltenerdacetat mehr bevorzugt eine Mischung aus Ytterbiumacetat und Erbiumacetat ist und ein Molverhältnis des Seltenerdacetats zu dem Zirkonium-/Hafniumacetylacetonat 1:4 beträgt;
wobei das Lösungsmittel vorzugsweise ein gemischtes Lösungsmittel aus Ölsäure und Octadecen ist, ein Volumenverhältnis von der Ölsäure zu dem Octadecen vorzugsweise 1:(0,5-3) beträgt, und mehr bevorzugt das Volumenverhältnis von der Ölsäure zu dem Octadecen 1:2 beträgt;
wobei ein Molverhältnis von dem Ammoniumfluorid zu dem Alkalimetallhydroxid 7:(6-12) beträgt, und das Molverhältnis von Ammoniumfluorid zu dem Alkalimetallhydroxid vorzugsweise 7:6 beträgt.

3. Verfahren nach Anspruch 1, wobei der Schritt S2 umfasst: in einer inerten Gasatmosphäre, Erwärmen der festen Mischung auf 120-160 °C und Inkubieren für 30-40 min,
und mehr bevorzugt, in einer inerten Atmosphäre, Erwärmen auf 140 °C, um die feste Mischung vollständig aufzulösen, und anschließendes natürliches Kühlen auf Raumtemperatur, um eine klare Lösung zu ergeben;
wobei Schritt S3 vorzugsweise umfasst: in einer inerten Gasatmosphäre, Erwärmen auf 50-70 °C und Inkubieren für 30 min, um Methanol zu entfernen, und Erwärmen auf 100-110 °C und Inkubieren für 10-20 min, um Wasser zu entfernen;
wobei der Schritt S4 vorzugsweise umfasst: in einer inerten Gasatmosphäre, kontinuierliches Erwärmen auf 300-310 °C und Inkubieren für 40-60 min;
wobei der Schritt S4 mehr bevorzugt umfasst: in einer inerten Gasatmosphäre, Erwärmen der Mischung auf 305 °C, Inkubieren für 40 min und natürliches Kühlen auf Raumtemperatur; vorzugsweise die Trennung aus Zentrifugation besteht.

## Revendications

1. Procédé de préparation d'un nanomatériau inorganique *in vivo* biodégradable de conversion ascendante, comprenant : le fait de soumettre un sel contenant un ion zirconium/hafnium, de l'acétate terreux rare, du fluorure d'ammonium et de l'hydroxyde de métal alcalin comme matières de départ à un processus de coprécipitation par solvant à haute température pour donner le nanomatériau de conversion ascendante de fluorure de métal alcalin à base de zirconium/hafnium dopé aux terres rares, dans lequel le nanomatériau inorganique *in vivo* biodégradable de conversion ascendante est dérivé d'un matériau de matrice de fluorure de métal alcalin à base de zirconium/hafnium dopé aux terres rares, dans lequel le matériau de matrice a une formule générale MₓT_{y}F_{x+4y}, contenant un nanocristal dopé aux terres rares ayant une formule développée générale MₓT_{y}F_{x+4y}:z% Ln, dans lequel M est un ou plusieurs parmi Li, Na et K ; T est Zr et/ou Hf ; Ln est un ou plusieurs choisis parmi Yb, Er, Tm, Ho, Gd, Eu, Tb, Sm, Dy, Ce et Nd ; 1 ≤ x ≤ 7 ; 1 ≤ y ≤ 6 ; a < z ≤ 50 ;
dans lequel le processus de coprécipitation par solvant à haute température comprend les étapes suivantes :
S1, peser, mélanger et ajouter de l'acétylacétonate de zirconium/hafnium et de l'acétate terreux rare à un solvant pour donner un mélange solide ;
S2, chauffer et incuber le mélange solide pour dissoudre les réactifs dans le mélange solide afin de donner une solution mélangée ;
S3, ajouter une solution de fluorure d'ammonium et d'hydroxyde de métal alcalin dans du méthanol à la solution mélangée obtenue à l'étape S2, puis chauffer et incuber pour éliminer le méthanol et l'eau ; et
S4, chauffer en continu et incuber, refroidir jusqu'à température ambiante, précipiter, séparer, laver et sécher pour donner le nanomatériau de conversion ascendante de fluorure de métal alcalin à base de zirconium/hafnium dopé aux terres rares.

2. Procédé selon la revendication 1, dans lequel le sel contenant un ion zirconium/hafnium est un ou plusieurs choisis parmi acétylacétonate de zirconium/hafnium, acétate de zirconium/hafnium, chlorure de zirconium/hafnium, nitrate de zirconium/hafnium et oxychlorure de zirconium/hafnium ; l'ion terreux rare dans l'acétate terreux rare est un ou plusieurs choisis parmi Yb, Er, Tm, Ho, Gd, Eu, Tb, Sm, Dy, Ce et Nd ;
de préférence, un rapport molaire de l'ion zirconium/hafnium sur le sel de métal terreux rare est (1-10):1, de préférence (2-5):1 ;
de préférence, l'acétate terreux rare est un mélange d'acétate d'ytterbium et d'acétate d'erbium, et un rapport molaire de l'acétate terreux rare sur l'acétylacétonate de zirconium/hafnium est 1:4 ;
de préférence, le solvant est un solvant mélangé d'acide oléique et d'octadécène, de préférence, un rapport volumique de l'acide oléique sur l'octadécène est 1:(0,5-3), et de manière davantage préférée, le rapport volumique de l'acide oléique sur l'octadécène est 1:2 ;
un rapport molaire du fluorure d'ammonium sur l'hydroxyde de métal alcalin est 7:(6-12), et de préférence, le rapport molaire du fluorure d'ammonium sur l'hydroxyde de métal alcalin est 7:6.

3. Procédé selon la revendication 1, dans lequel l'étape S2 comprend : dans une atmosphère de gaz inerte, le chauffage du mélange solide jusqu'à 120-160 °C et l'incubation pendant 30-40 min,
et de manière davantage préférée, dans une atmosphère inerte, le chauffage jusqu'à 140 °C pour dissoudre complètement le mélange solide, puis le refroidissement naturel jusqu'à température ambiante pour donner une solution limpide ;
de préférence, l'étape S3 comprend : dans une atmosphère de gaz inerte, le chauffage jusqu'à 50-70 °C et l'incubation pendant 30 min pour éliminer le méthanol, et le chauffage jusqu'à 100-110 °C et l'incubation pendant 10-20 min pour éliminer l'eau ;
de préférence, l'étape S4 comprend : dans une atmosphère de gaz inerte, le chauffage en continu à 300-310 °C et l'incubation pendant 40-60 min ;
de manière davantage préférée, l'étape S4 comprend : dans une atmosphère de gaz inerte, le chauffage du mélange jusqu'à 305 °C, l'incubation pendant 40 min et le refroidissement naturel jusqu'à température ambiante ; de préférence, la séparation est une centrifugation.
